# EUROPEAN PATENT APPLICATION

(11) **EP 3 321 362 A1**
(43) Date of publication of application: **16.05.2018**
(21) Application number: 16306475.1
(22) Date of filing: 10.11.2016
(51) Int. Cl.: C12N 15/117

(54) **TLR3 AGONIST FOR USE FOR INDUCING APOPTOSIS IN SENESCENT CANCER CELLS**

(71) Applicant: Centre Léon Bérard, 69008 Lyon (FR)
(72) Inventor: LEBECQUE, Serge, 69380 CIVRIEUX D'AZERGUES (FR); RENNO, Toufic, 69380 CIVRIEUX D'AZERGUES (FR); VANBERVLIET, Béatrice, 69008 LYON (FR); ESTORNES, Yann, 69100 VILLEURBANNE (FR)
(74) Representative: Lavoix

(57) **Abstract**

The present invention relates to a TLR3 agonist for use for inducing apoptosis in senescent cancer cells. In particular, it relates to a TLR3 agonist for use for the treatment of cancer, optionally in combination with a chemotherapeutic agent or with radiotherapy. It further relates to a pharmaceutical composition comprising a TLR3 agonist and a chemotherapeutic agent.

## Description

The present invention relates to a TLR3 agonist for use for inducing apoptosis in senescent cancer cells. In particular, it relates to a TLR3 agonist for use for the treatment of cancer, optionally in combination with a chemotherapeutic agent or with radiotherapy. It further relates to a pharmaceutical composition comprising a TLR3 agonist and a chemotherapeutic agent.

### Background of the invention

As a result of ageing, normal cells tend to undergo replicative senescence, meaning that they become essentially irreversibly arrested in the G1 phase of the cell cycle and no longer able to divide despite remaining viable and metabolically active for long periods of time. Senescence can be induced in cancer cells either by oncogenic stress or by therapy (i.e. genotoxic stress). Oncogene, chemotherapeutic agents and ionizing radiations can indeed lead to prolonged cell cycle arrest in cancer cells. In some cases, senescence appears to occur spontaneously.

Senescence, which acts as defense mechanisms against cell transformation, exerts pro-tumorigenic activities through the senescent cell's secretome that promotes tumor-specific features, such as cellular proliferation, epithelial-mesenchymal transition and invasiveness.

However, elimination of cancer senescent cells is a difficult task as their resting state protects them from classical cancer therapies that target highly proliferating cells.

The ability of senescent cancer cells to resist to most of the current cancer therapies, the large panel of inflammatory chemokines and cytokines (senescence associated secretome) they secrete, and their ability to occasionally re-enter the cell cycle altogether represent a serious medical threat.

There is thus a need to find out a way to eliminate senescent cancer cells.

### Description of the invention

The inventors of the present invention have now shown that cancer cells expressing TLR3 remain sensitive to TLR3-triggered apoptosis after undergoing senescence. They thus found a new class of molecules that can eliminate senescent cancer cells, that can eliminate the risk of cancer senescent cells re-entry into the cell cycle, and that can eliminate the side effects associated with senescent cancer cells secretome.

The present invention thus relates to a TLR3 agonist for use for inducing apoptosis in senescent cancer cells.

It also relates to a TLR3 agonist for use as mentioned above for the treatment of cancer.

It further relates to a TLR3 agonist for use as mentioned above, to improve the general health condition of a cancer patient.

In particular, the general health condition of the cancer patient is improved by reducing and/or eliminating the side effects associated with senescent cancer cells secretome.

The present invention also relates to a TLR3 agonist for use as mentioned above, in combination with a chemotherapeutic agent or with radiotherapy.

The present invention further relates to a pharmaceutical composition comprising a TLR3 agonist and a chemotherapeutic.

In one embodiment, the invention relates to a method for inducing apoptosis in senescent cancer cells, comprising the administration, in a patient in need thereof, of a therapeutically effective amount of a TLR3 agonist.

In particular, said method is for the treatment of cancer.

By "general health condition of a cancer patient" is meant the state of physical and mental tiredness of said cancer patient. This general health condition of the cancer patient is determined by the use of clinical scores (i.e. Karnofsky' score or Zubrod' score).

The Karnofsky score runs from 100 to 0, where 100 is "perfect" health and 0 is death. Practitioners occasionally assign performance scores in between standard intervals of 10. This scoring system is named after Dr. David A. Karnofsky, who described the scale with Dr. Walter H. Abelmann, Dr. Lloyd F. Craver, and Dr. Joseph H. Burchenal in 1948. The primary purpose of its development was to allow physicians to evaluate a patient's ability to survive chemotherapy for cancer.
- 100 - Normal; no complaints; no evidence of disease.
- 90 - Able to carry on normal activity; minor signs or symptoms of disease.
- 80 - Normal activity with effort; some signs or symptoms of disease.
- 70 - Cares for self; unable to carry on normal activity or to do active work.
- 60 - Requires occasional assistance, but is able to care for most of their personal needs.
- 50 - Requires considerable assistance and frequent medical care.
- 40 - Disabled; requires special care and assistance.
- 30 - Severely disabled; hospital admission is indicated although death not imminent.
- 20 - Very sick; hospital admission necessary; active supportive treatment necessary.
- 10 - Moribund; fatal processes progressing rapidly.
- 0 - Dead

The Eastern Cooperative Oncology Group (ECOG) score (published by Oken *et al.* in 1982), also called the WHO or Zubrod score (after C. Gordon Zubrod), runs from 0 to 5, with 0 denoting perfect health and 5 death. Its advantage over the Karnofsky scale lies in its simplicity.
- 0 - Asymptomatic (Fully active, able to carry on all predisease activities without restriction)
- 1 - Symptomatic but completely ambulatory (Restricted in physically strenuous activity but ambulatory and able to carry out work of a light or sedentary nature. For example, light housework, office work)
- 2 - Symptomatic, <50% in bed during the day (Ambulatory and capable of all self care but unable to carry out any work activities. Up and about more than 50% of waking hours)
- 3 - Symptomatic, >50% in bed, but not bedbound (Capable of only limited self-care, confined to bed or chair 50% or more of waking hours)
- 4 - Bedbound (Completely disabled. Cannot carry on any self-care. Totally confined to bed or chair)
- 5 - Death

By "senescent cancer cells secretome" is meant the panel of inflammatory chemokines and cytokines that are secreted by senescent cancer cells. They include among others IL-1α, IL-1β, IL-6, IL-8, TGFβ, EGFR, CCL1, CCL2, CCL3, CXCL1, CXCL5, MMP3, MMP5.

By "TLR3 agonist" is meant Toll-like receptor 3 agonist. TLR3 agonists are well known by the man skilled in the art. It refers to an affinity agent (i.e., a molecule that binds a target molecule) capable of activating a TLR3 polypeptide to induce a full or partial receptor-mediated response. For example, an agonist of TLR3 induces TLR3 dimerization/oligomerization and triggers TLR3-mediated signaling, either directly or indirectly. A TLR3 agonist, as used herein may, but is not required to, bind a TLR3 polypeptide, and may or may not interact directly with the TLR3 polypeptide. They include double stranded ribonucleic acid (dsRNA) such as: Poly(A:U) for Polyadenylic-polyuridylic acid, Poly(I:C) for Polyinosine-polycytidylic acid, Poly(ICLC) (Hiltonol^{®}) , Polyl:PolyC12U (Ampligen^{®}) or RGIC dsRNA such as RGIC 100.1 (Riboxx^{®}).

Such TLR3 agonists are for example described in the patent US8409813, in particular in columns nine to twenty two, in the patent EP2281043, in the patent application WO2015/091578 and in the patent application WO2008/109083. In particular, the RGIC100.1 is described in examples 1 and 2 of WO2015/091578.

In particular a TLR3 agonist according to the invention is a double strand ribonucleic acid (dsRNA) such as Polyinosinic:polycytidylic acid (Poly(I:C)) or RGIC dsRNA.

By "senescent cancer cells" is meant cells no longer able to divide despite remaining viable and metabolically active for long periods of time. Senescence can be induced in cancer cells either by oncogenic stress or by therapy (i.e. genotoxic stress) or can arise spontaneously.

In particular, the senescent cancer cells are senescent cancer cells induced by stress. These stresses can be of genetic types (oncogenic activation), metabolic (oxidative stress) or be environmental (cytotoxic drugs, ionizing radiations).

More particularly, the senescent cancer cells are chemotherapy-induced senescent cancer cells, radiotherapy-induced senescent cancer cells or spontaneous senescent cancer cells, and even more particularly chemotherapy-induced senescent cancer cells.

By "spontaneous senescent cancer cells" is meant cancer cells for which the senescence appears to occur spontaneously probably as a response to the constraints imposed upon cancer cells by unfavorable micro-environment.

By "cancer" is meant the growth, division or proliferation of abnormal cells in the body. In particular, cancers covered are those that expressed TLR3. The determination of TLR3 expression in cancer cells is well within the ability of the man skilled in the art and can be measured by any method available to the man skilled in the art such as immunohistochemistry, Western Blot, or quantitative PCR (for example by using the LightCycler^{®} System of Roche Molecular Diagnostics), etc. Still particularly, cancers covered by the present invention are chosen from: epithelial cancers such as Small-cell Lung cancers, Non-Small-Cell Lung cancer, lung adenocarcinomas, hepatocarcinoma, Head and Neck, ovarian, renal, bladder, prostate, breast, cervix, pancreas, esophageal, gastric, small intestine, colon, or melanoma cancers and mesenchymal cancers such as mesothelioma or sarcoma cancer, and more particularly Non-Small-Cell Lung cancer.

In particular the senescent cancer cells according to the invention are chosen from epithelial senescent cancer cells such as Small-cell Lung, Non-Small-Cell Lung, lung adenocarcinomas, hepatocarcinoma, Head and Neck, ovarian, renal, bladder, prostate, breast, cervix, pancreas, esophageal, gastric, small intestine, colon, or melanoma senescent cancer cells and mesenchymal senescent cancer cells such as mesothelioma or sarcoma senescent cancer cells, and more particularly Non-Small-Cell Lung senescent cancer cells.

By "chemotherapeutic agent" is meant a cytotoxic agent that is known to be of use in chemotherapy for cancer, in the context of the invention, for epithelial cancer such as Small-cell Lung, Non-Small-Cell Lung, lung adenocarcinomas, hepatocarcinoma, Head and Neck, ovarian, renal, bladder, prostate, breast, cervix, pancreas, esophageal, gastric, small intestine, colon, or melanoma cancers and for mesenchymal cancers such as mesothelioma or sarcoma cancers. It encompasses standard chemotherapeutic agents such as paclitaxel and non-conventional chemotherapeutic agents such as irinotecan. Topoisomerase inhibitors, gemcitabine, 5-Fluorouracil, oxaliplatin and doxorubicin can be cited as examples.

In particular, a chemotherapeutic agent according to the invention is chosen from paclitaxel, topoisomerase inhibitors, gemcitabine, 5-Fluorouracil, oxaliplatin and doxorubicin, and more particularly is paclitaxel. These compounds are well known by the man skilled in the art.

By "radiotherapy", "radiation therapy", or "radiation oncology", often abbreviated RT, RTx, or XRT, is meant the medical use of ionizing radiation, generally as part of cancer treatment to control or kill malignant cells.

In particular, the radiotherapy according to the invention is chosen from gamma-rays or X-rays.

As used herein, the term "subject" or "patient" refers to a warm-blooded animal such as a mammal, animal or human, in particular a human, who is afflicted with, or has the potential to be afflicted with one or more diseases and conditions described herein.

The terms "treat", "treating", "treated" or "treatment", as used herein, refer to therapeutic treatment wherein the object is to eliminate or lessen symptoms. Beneficial or desired clinical results include, but are not limited to, elimination of symptoms, alleviation of symptoms, diminishment of extent of condition, stabilized (i.e., not worsening) state of condition, delay or slowing of progression of the condition, to the prevention of the onset, recurrence or spread of a disease or disorder, or of one or more symptoms thereof. In certain embodiments, the terms refer to the treatment with or administration of a compound provided herein prior to the onset of symptoms. The terms encompass the inhibition or reduction of a symptom of the particular disease. Subjects with familial history of a disease in particular are candidates for treatment regimens in certain embodiments. Also, subjects in whom a genetic disposition for the particular disease has been shown are candidates for treatment regimens in certain embodiments. In addition, subjects who have a history of recurring symptoms are also potential candidates for the treatment. In this regard, the term "treatment" may be interchangeably used with the term "prophylactic treatment."

The TLR3 agonist according to the invention as well as the chemotherapeutic agent, can be formulated into pharmaceutical compositions by admixture with one or more pharmaceutically acceptable excipients.

As used herein, a "pharmaceutically acceptable excipient" refers to molecular entities and compositions that do not produce an adverse, allergic or other untoward reaction when administered to a mammal, especially a human, as appropriate. A pharmaceutically acceptable excipient refers to a non-toxic solid, semi-solid or liquid filler, diluent, encapsulating material or formulation auxiliary of any type.

Such compositions may be prepared for use in oral administration, particularly in the form of tablets or capsules, in particular orodispersible (lyoc) tablets; or parenteral administration, particularly in the form of liquid solutions, suspensions or emulsions.

It may be prepared by any of the methods well known in the pharmaceutical art, for example, as described in Remington: The Science and Practice of Pharmacy, 20th ed.; Gennaro, A. R., Ed.; Lippincott Williams & Wilkins: Philadelphia, PA, 2000. Pharmaceutically compatible binding agents and/or adjuvant materials can be included as part of the composition. Oral compositions will generally include an inert diluent carrier or an edible carrier. They can be administered in unit dose forms, wherein the term "unit dose" means a single dose which is capable of being administered to a patient, and which can be readily handled and packaged, remaining as a physically and chemically stable unit dose comprising either the active compound itself, or as a pharmaceutically acceptable composition, as described hereinafter.

The tablets, pills, powders, capsules, troches and the like can contain one or more of any of the following ingredients, or compounds of a similar nature: a binder such as microcrystalline cellulose, or gum tragacanth; a diluent such as starch or lactose; a disintegrant such as starch and cellulose derivatives; a lubricant such as magnesium stearate; a glidant such as colloidal silicon dioxide; a sweetening agent such as sucrose or saccharin; or a flavoring agent such as peppermint, or methyl salicylate. Capsules can be in the form of a hard capsule or soft capsule, which are generally made from gelatin blends optionally blended with plasticizers, as well as a starch capsule. In addition, dosage unit forms can contain various other materials that modify the physical form of the dosage unit, for example, coatings of sugar, shellac, or enteric agents. Other oral dosage forms syrup or elixir may contain sweetening agents, preservatives, dyes, colorings, and flavorings. In addition, the active compounds may be incorporated into fast dissolve, modified-release or sustained-release preparations and formulations, and wherein such sustained-release formulations are preferably bi-modal.

Liquid preparations, in particular for intravenous or oral administration, include sterile aqueous or non-aqueous solutions, suspensions, and emulsions. The liquid compositions may also include binders, buffers, preservatives, chelating agents, sweetening, flavoring and coloring agents, and the like. Non-aqueous solvents include alcohols, propylene glycol, polyethylene glycol, acrylate copolymers, vegetable oils such as olive oil, and organic esters such as ethyl oleate. Aqueous carriers include mixtures of alcohols and water, hydrogels, buffered media, and saline. In particular, biocompatible, biodegradable lactide polymer, lactide/glycolide copolymer, or polyoxyethylene-polyoxypropylene copolymers may be useful excipients to control the release of the active compounds. Intravenous vehicles can include fluid and nutrient replenishers, electrolyte replenishers, such as those based on Ringer's dextrose, and the like.

Examples of modes of administration include parenteral e.g. subcutaneous, intramuscular, intravenous, intradermal, as well as oral administration.

TLR3 agonists, chemotherapeutic agents, and pharmaceutical compositions of the invention may be prepared by a variety of synthetic routes. The reagents and starting materials are commercially available, or readily synthesized by well-known techniques by one of ordinary skill in the arts.

The identification of the subjects who are in need of treatment of herein-described diseases and conditions is well within the ability and knowledge of one skilled in the art. A clinician skilled in the art can readily identify, by the use of clinical tests, physical examination and medical/family history, those subjects who are in need of such treatment.

A therapeutically effective amount can be readily determined by the attending diagnostician, as one skilled in the art, by the use of conventional techniques and by observing results obtained under analogous circumstances. In determining the therapeutically effective amount, a number of factors are considered by the attending diagnostician, including, but not limited to: the species of subject; its sex, size, weight, age, and general health; the specific disease involved; the expression of TLR3 (measured by any method available to the diagnostician such as immunohistochemistry, quantitative PCR, Western Blot, etc), the degree of involvement or the severity of the disease; the response of the individual subject; the particular compound administered; the mode of administration; the bioavailability characteristic of the preparation administered; the dose regimen selected; the use of concomitant medication; and other relevant circumstances.

The amount of TLR3 agonist of the invention and of the chemotherapeutic agent, which is required to achieve the desired biological effect, will vary depending upon a number of factors, including the dosage of the drug to be administered, the chemical characteristics (e.g. hydrophobicity) of the compounds employed, the potency of the compounds, the type of disease, the diseased state of the patient, and the route of administration.

In particular, the doses of TLR3 agonist according to the invention that can be administered are between 0,1 mg/kg and 10 mg/kg.

Each compound of the combinations or pharmaceutical compositions according to the invention can be administered separately, sequentially or simultaneously.

Accordingly, in a further embodiment of the methods, combination, pharmaceutical composition or use according to the invention, a TLR3 agonist is administered in combination with a chemotherapeutic agent in a combined preparation for simultaneous, separate, or sequential use.

If administered sequentially , the TLR3 agonist should preferably be administered after the administration of the chemotherapeutic agent.

If administered separately, they can be administered by the same or different modes of administration. Examples of modes of administration include parenteral (e.g., subcutaneous, intramuscular, intraorbital, intracapsular, intraspinal, intrasternal, intravenous, intradermal, intraperitoneal, intraportal, intra-arterial, intrathecal, transmucosal, intra-articular, and intrapleural), transdermal (e.g., topical), epidural, and mucosal (e.g. intranasal) injection or infusion, as well as oral, inhalation, pulmonary, and rectal administration.

When the TLR3 agonist according to the invention is used in combination with radiotherapy, radiotherapy can be carried out before the administration of the TLR3 agonist according to the invention or the TLR3 agonist can be administered concomitantly to the irradiation.

In the scope of the present invention, it has to be understood that "a TLR3 agonist for use" is equivalent to "the use of a TLR3 agonist" and in particular that "a TLR3 agonist for use in the treatment of" is equivalent to "the use of a TLR3 agonist for the treatment of" and to "the use of a TLR3 agonist for the manufacture of a medicament intended for the treatment of".

The invention will be further illustrated by the following figures and examples.

### FIGURES

**Figure 1****:** NCI-H292 cells were treated for 1, 3 and 7 days with solvent (A-C) or with paclitaxel (4nM) (D-F). Cells are viewed by contrast phase microscopy. Bar size=15µm.
**Figure 2****:** NCI-H292 cells cultured for 7 days with paclitaxel (4 nM) have been treated to detect senescence-associated beta-galactosidase activity and analyzed by contrast phase microscopy. Bar represent 15 µm.
**Figure 3****:** NCI-H292 cells have been cultured for 1, 3 or 7 days without (control) or with 4 nM of paclitaxel. IL-8 secretion during the last 24H of culture has been measured by Elisa.
**Figure 4****:** Analysis by flow cytometry after intracellular staining with control IgG1 (first pic) or anti-hTLR3 mAb TLR3.1 (second pic) of NCI-H292 cells cultured for 7 days without (A) or with paclitaxel at 4nM (B).
**Figure 5****:** Chemotherapy-Treated Cells are more sensitive to TLR3-triggered apoptosis when they have undergone paclitaxel-induced senescence.
**Figure 6****:** Radiation-Induced Senescent Cells are more sensitive to TLR3-triggered apoptosis when they have undergone radiation-induced senescence.
**Figure 7****:** Oxydative Stress-induced Senescent Cells are more sensitive to TLR3-triggered apoptosis when they have undergone oxidative stress-induced senescence.

### EXAMPLES

### I- Chemotherapy-Treated Cells

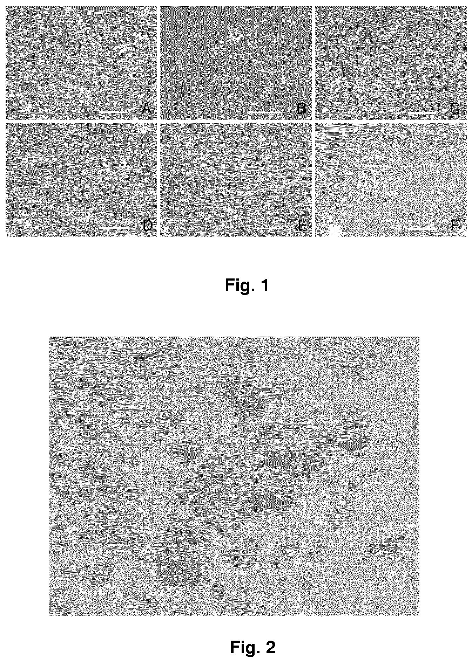

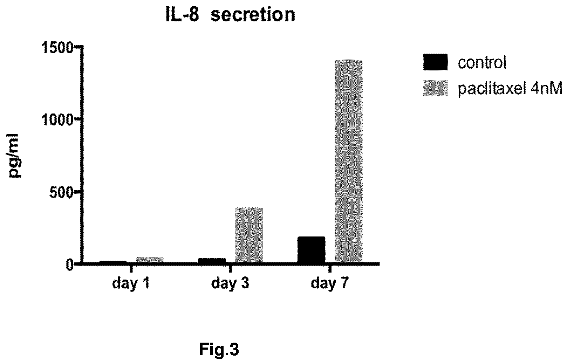

NCI-H292 human Non-Small-Cell Cancer Cells were plated at 5000 cells/cm² in a 6-well plate in RPMI supplemented with 10% FCS and 1%Hepes/1%NAPy/PS. They were treated after 16-24 hours of plating and for 7 to 10 days with 4nM paclitaxel (Sigma) or with DMSO ("solvent"), with a change of medium on day 4. The following tests were then performed to ascertain the Paclitaxel-Treated Cells' senescence status.
1) An inverted phase-contrast light microscope was used at 40X magnification to evaluate morphology of the Paclitaxel-Treated Cells. Observed changes included a stop in cell division and enlargement in size, which are features associated with senescence (Figure 1).
2) The senescence-associated β-galactosidase [SA-β-GAL] activity was measured in the Paclitaxel-Treated Cells described above using the Senescence beta-galactosidase Staining Kit (Cell Signaling Technology) (Figure 2).
3) The secretion of IL-8 as part of the senescence-associated secretome (Tchkonia et al., 2013; J Clin Invest. 123(3):966-72) was quantified in the culture supernatant of the Paclitaxel-Treated Cells using an ELISA kit (Biolegend Kit huIL8), and read on a TECAN microplate reader). Large amounts of IL-8 were found in the supernatant of Paclitaxel-Treated Cells in a time-dependent manner (Figure 3).

The results described in Figures 1-3 indicated that the Paclitaxel-Treated Cells have become senescent.

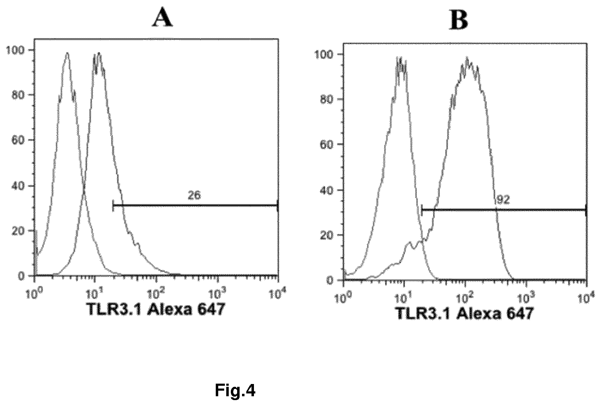

To ascertain that TLR3 expression was not lost upon senescence, Paclitaxel-Treated Cells were harvested, fixed and permeabilized with the Cytoperm/Cytofix kit (Becton Dickinson), then stained at 4°C for TLR3 (anti-TLR3.1 antibody (Dendritics) + goat antimouse Alexa 647) and analyzed on a Becton Disckison FACSCalibur flow cytometer. Surprisingly, the expression of TLR3 by NCI-H292 cells had increased after 7 days of culture in the presence of paclitaxel (4nM) (Figure 4).

After having shown that Paclitaxel-Treated Cells were senescent and had a strong expression of TLR3, we measured their susceptibility to TLR3-triggered apoptosis. TLR3 WT and TLR3 KO NCI-H292 untreated or Paclitaxel-Treated Cells were incubated for 24 hours with 4 µg/ml Poly(I:C) (Invivogen), then cells were stained at 4°C with Annexin V-FITC and PI (Biolegend) and analyzed on a Becton Disckison FACSCalibur flow cytometer.

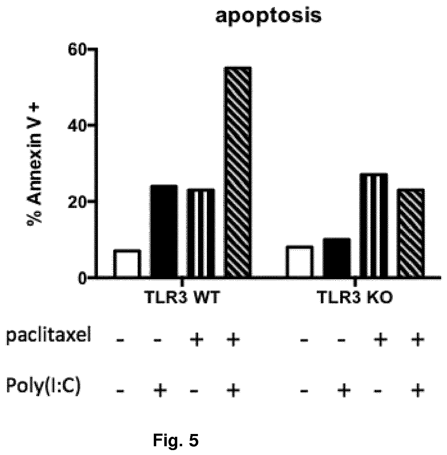

Figure 5 shows that Paclitaxel-Treated Cells are more sensitive to TLR3-triggered apoptosis when they have undergone paclitaxel-induced senescence.

Apoptosis induction by double-stranded RNA is not limited to cancer cells that were made senescent by Paclitaxel. Indeed, it was also showed by the inventors that Poly(I:C) induces apoptosis in cancer cells that were made senescent by irradiation or by oxidative stress (H₂O₂).

### II- Radiation-Induced Senescent Cells

NCI-H292 human Non-Small-Cell Cancer Cells were plated at 5000 cells/cm² in a 6-well plate in RPMI supplemented with 10% FCS and 1%Hepes/1%NAPy/PS. They were irradiated with 8 Grey 16 hours after plating, and the medium was changed right after irradiation.

Radiation-Induced Senescent Cells' susceptibility to TLR3-triggered apoptosis was measured. Untreated or Radiation-Induced Senescent WT and TLR3 KO NCI-H292 Cells were incubated for 24 hours with 10 µg/ml Poly(I:C) (Invivogen), then cells were stained at 4°C with Annexin V-FITC PI (Biolegend) and analyzed on a Becton Disckison FACSCalibur flow cytometer.

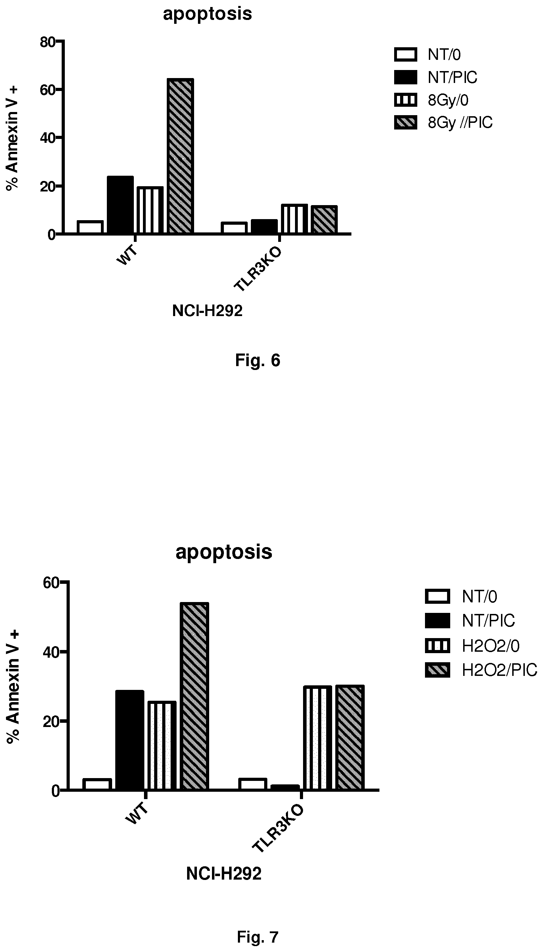

Figure 6 shows that Radiation-Induced Senescent Cells are more sensitive to TLR3-triggered apoptosis when they have undergone radiation-induced senescence.

### III- Oxydative Stress-induced Senescent Cells

NCI-H292 human Non-Small-Cell Cancer Cells were plated at 5000 cells/cm² in a 6-well plate in RPMI supplemented with 10% FCS and 1%Hepes/1%NAPy/PS. They were treated with 300 µM H₂O₂ 16 hours after plating, and the medium was changed 1 hour after H₂O₂ treatment.

Oxydative Stress-induced Senescent Cells' susceptibility to TLR3-triggered apoptosis was measured. Untreated or Oxydative Stress-induced Senescent WT and TLR3 KO NCI-H292 Cells were incubated for 24 hours with 10 µg/ml Poly(I:C) (Invivogen), then cells were stained at 4°C with Annexin V-FITC (Biolegend) and analyzed on a Becton Disckison FACSCalibur flow cytometer.

Figure 7 shows that Oxydative Stress-induced Senescent Cells are more sensitive to TLR3-triggered apoptosis when they have undergone oxidative stress-induced senescence.

## Claims

1. A TLR3 agonist for use for inducing apoptosis in senescent cancer cells.

2. A TLR3 agonist for use according to claim 1, wherein the senescent cancer cells are senescent cancer cells induced by stress.

3. A TLR3 agonist for use according to claim 1 or 2, wherein the senescent cancer cells are chemotherapy-induced senescent cancer cells, radiotherapy-induced senescent cancer cells or spontaneous senescent cancer cells.

4. A TLR3 agonist for use according to any of the preceding claims, for use for the treatment of cancer.

5. A TLR3 agonist for use according to any of the preceding claims, in combination with a chemotherapeutic agent or with radiotherapy.

6. A TLR3 agonist for use according to any of the preceding claims, for use to improve the general health condition of a cancer patient.

7. A TLR3 agonist for use according to claim 6, wherein the general condition of the cancer patient is improved by reducing and/or eliminating the side effects associated with senescent cancer cells secretome.

8. A TLR3 agonist for use according to any of the preceding claims, wherein the senescent cancer cells are chosen from epithelial senescent cancer cells such as Small-cell Lung, Non-Small-Cell Lung, lung adenocarcinomas hepatocarcinoma, Head and Neck, ovarian, renal, bladder, prostate, breast, pancreas, esophageal, gastric, small intestine, colon, or melanoma senescent cancer cells, cervix cancer and mesenchymal senescent cancer cells such as mesothelioma or sarcoma senescent cancer cells.

9. A pharmaceutical composition comprising a TLR3 agonist and a chemotherapeutic agent.

10. A TLR3 agonist for use according to any of claims 5 to 8 or a pharmaceutical composition according to claim 9, wherein the chemotherapeutic agent is chosen from paclitaxel, topoisomerase inhibitors, gemcitabine, 5-Fluorouracil, oxaliplatin and doxorubicin.

11. A TLR3 agonist for use according to any of claims 5 to 8, wherein the radiotherapy is chosen from gamma-rays or X-rays.

12. A TLR3 agonist for use according to any of claims 1 to 8 and 10 to 11 or a pharmaceutical composition according to any of claims 9 to 10, wherein said TLR3 agonist is chosen from a double strand ribonucleic acid (dsRNA) such as Polyinosinic:polycytidylic acid (Poly(I:C)) or RGIC dsRNA.
